# EUROPEAN PATENT APPLICATION

(11) **EP 4 610 988 A1**
(43) Date of publication of application: **03.09.2025**
(21) Application number: 24215798.0
(22) Date of filing: 27.11.2024
(51) Int. Cl.: G16C 60/00, G16C 20/30, G16C 20/70

(54) **SYSTEM, METHOD AND PROGRAM FOR PREDICTING PROPERTIES OF MATERIAL HAVING MULTI-PHASE USING ARTIFICIAL INTELLIGENCE**

(30) Priority: 28.11.2023 KR 20230168608
(71) Applicant: LG Management Development Institute, Seoul 07336 (KR)
(72) Inventor: Park, Changyoung, 07982 Seoul (KR); Lee, Jaewan, 07792 Seoul (KR); Yang, Hongjun, 07788 Seoul (KR); Han, Sehui, 07665 Seoul (KR)
(74) Representative: BCKIP Part mbB

(57) **Abstract**

A system, method, and program predict the properties of a material having a multi-phase. The system for implementing an AI model of predicting the properties of a material having a multi-phase includes memory configured to store instructions that are executable; and one or more processors configured to execute the instructions to perform operations comprising: inputting first material information, which includes information regarding the material, into a first AI model to output first feature data; inputting first phase information, which includes information regarding a first phase of the material, into a second AI model to output first phase feature data; and inputting second phase information, which includes information regarding a second phase of the material, into the second AI model to output second phase feature data; and wherein the first feature data includes information regarding the properties of the material according to the multi-phase of the material.

## Description

This application claims the priority to Korean Patent Application No. 10-2023-0168608, filed on November 28, 2023.

### TECHNICAL FIELD

The present disclosure generally relates to a system, method, and program for predicting the properties of a material having a multi-phase, and more specifically, to a system, method, and program for predicting the properties of a material having a multi-phase using artificial intelligence.

### BACKGROUND

Recently, with the diversification of consumer demands and trends, as well as the various ways of product development, the development of materials that may be used in manufacturing products has been actively progressing. These materials may impact the properties of products, and some properties of the materials (e.g., composition) may become key factors in determining the properties of the products manufactured. Therefore, in order to efficiently develop and mass-produce materials used in manufacturing products, the prediction and analysis of the properties of that material may be needed.

Traditionally, in order to verify the properties of a product manufactured according to the properties of the material, a method of developing various materials, verifying the properties of each material, and then test-applying them to the final product to verify the properties has been used. These traditional methods required the process of actually manufacturing various materials, verifying their properties, and then manufacturing a product using the materials again, and then verifying the properties of that product. However, this method requires a lot of cost and time for material and property development, and it is also difficult to find a material with optimal properties.

As a method to improve this traditional way, a method using artificial intelligence to predict the properties of materials was disclosed in Korean Patent Application Publication No. 10-2022-0060346.

The conventional method of predicting the properties of a material using artificial intelligence invented assumed that the material has one phase, extracted the representative of that material, and used this to predict the properties of that material.

However, when actually using a material, there are cases where it changes into various phases depending on the environment, usage method, etc., and in such cases, if a prediction method that assumes a conventional single phase is used, the prediction accuracy was low, making it difficult to utilize the prediction results in the development of actual materials and products.

For example, the cathode of a lithium-ion secondary battery includes a cathode material containing Li, and when the lithium-ion secondary battery is charged and discharged, the Li of the cathode material is absorbed and released in the form of Li ions (Li+). Since the Li atoms in the cathode material escape during charging in the principle of these lithium-ion secondary batteries, the composition ratio of Li contained in the cathode material in the charged state and the composition ratio of Li contained in the cathode material in the discharged state are different, so the cathode material has multi-phases during actual use.

However, the conventional method of predicting the properties of materials using artificial intelligence assumes that they have one phase, so the properties are predicted based on the Li composition at the time of manufacturing the cathode material, and the properties of the product are predicted using these. However, when the cathode material is actually used in a lithium-ion secondary battery, it has multi-phase because the Li composition changes, so with the conventional method of predicting using single-phase as above, the prediction results for the material properties and product properties are different from the actual results, thereby reducing the usability of the artificial intelligence prediction method.

### SUMMARY

Some embodiments of the present disclosure may provide a system, method, and program that uses artificial intelligence to secure representative for the multiple phases that a material may have, and uses these to more accurately predict the properties of a material having the multiple phases.

The problem to be solved by the present invention is not limited to the problem mentioned above, and other problems not mentioned will be clearly understood by those skilled in the art from the description below.

A system for implementing an AI model of predicting the properties of a material having a multiphase according to one aspect of the present disclosure includes: at least one processor; and at least one memory that stores command or information that allows the at least one processor to perform operations, wherein the operations performed by the commands or information include: inputting first material information, which includes information regarding the material, into a first AI model to output first feature data; inputting first phase information, which includes information regarding the first phase of the material, into a second AI model to output first phase feature data; and inputting second phase information, which includes information regarding a second phase of the material, different from the first phase, into the second AI model to output second phase feature data; and wherein the first feature data includes information regarding the properties of the material according to the phases of the material.

The operations performed by the commands or information may further include inputting the first feature data, the first phase feature data, and the second phase feature data into a third AI model.

The operations performed by the commands or information may further include: summing the first phase feature data with the first feature data and inputting them into the third AI model to output first prediction data; and summing the second phase feature data with the first feature data and inputting them into the third AI model to output second prediction data.

The system may further include a calculation unit that receives the first and second prediction data and outputs a target.

The operations performed by the commands or information further include summing the first feature data, the first phase feature data, and the second phase feature data and inputting them into the third AI model.

In embodiments, the first feature data may include a class token form.

In other embodiments, the system may further include a transformation unit that receives concatenated embed and outputs transformed data, wherein the concatenated embed may include: the first feature data; second material information including information regarding the material; and at least one of information regarding a substance or device including the material, wherein the second material information may be different from the first material information.

The transformed data may include second feature data, the second feature data may include information regarding the first feature data, and the second feature data may further include at least one of the second material information and information regarding the substance or device including the material.

The operations performed by the commands or information may further include inputting the transformed data into a fourth AI model to output a predicted value.

The predicted value may include a property value of the substance or device including the material.

In embodiments, at least one of the first feature data and the second feature data may be a feature vector.

A method for predicting the properties of a material having a multiphase through an AI model according to another aspect of the present disclosure, the method being performed by at least one processor may include: inputting first material information, which includes information regarding the material, into a first AI model to output first feature data; inputting first phase information, which includes information regarding the first phase of the material, into a second AI model to output first phase feature data; and inputting second phase information, which includes information regarding a second phase of the material, different from the first phase, into the second AI model to output second phase feature data; wherein the first AI model and second AI model may be executed or learned by the processor, and wherein the first feature data may include information regarding the properties of the material according to phases of the material.

The method may further include inputting the first feature data, the first phase feature data, and the second phase feature data into a third AI model.

The method may further include: inputting concatenated embed into a transformation unit and outputting transformed data by the processor, wherein the concatenated embed may include: the first feature data; second material information including information regarding the material; and at least one of information regarding a substance or device including the material, wherein the second material information may be different from the first material information.

A program according to another aspect of the present disclosure, coupled with a computer, may be one stored on a computer-readable medium for executing the method of predicting the properties of a material having a multiphase through an AI model according to the embodiments of the present disclosure.

A system for implementing an AI model of predicting the properties of a material having a multiphase according to another aspect of the present disclosure, may include: at least one processor; and at least one memory that stores command or information that allows the at least one processor to perform operations, wherein the operations performed by the commands or information may include: inputting an embed, which includes first feature data regarding the material, first material information of the material, and at least one of information regarding a substance or device including the material, into a transformation unit to output transformed data; and inputting the transformed data into a fourth AI model to output a predicted value, wherein the first feature data may include information regarding the properties of the material according to the phases of the material.

According to certain embodiments of the present disclosure, since an AI model configured to learn by inputting material information and multiple phase information is utilized, the learned feature data already includes information about the phases of the material, and thus the feature data may be utilized as input data as a representative vector of the material without obtaining or inputting information on multiple phases, when predicting the properties of a material and/or the properties of a substance or device including the material.

In addition, a method of predicting the properties of the material and/or the substance or device including the material according to certain embodiments of the present disclosure may use feature data derived through a pre-learned AI model as an input representative vector, and since the feature data already includes information regarding to the properties according to the phase of the material, even when predicting the properties of a material having a multi-phase, properties prediction may be performed accurately without separately inputting phase information.

In addition, according to some embodiments of the present disclosure, prediction of properties of material may be performed accurately with only information input into the feature data, so accurate prediction of properties can be accomplished with only a small amount of information.

The effects of the present invention are not limited to the effects mentioned above, and other effects that have not been mentioned will be clearly understood by those skilled in the art from the following description.

### BRIEF DESCRIPTION OF THE DRAWINGS

FIG. 1 is a schematic diagram of a system for implementing a method of obtaining feature data for material composition information based on artificial intelligence according to an embodiment of the present disclosure.
FIG. 2 is a block diagram for explaining the configuration of a device that performs a method of obtaining feature data for material composition information based on artificial intelligence according to an embodiment of the present disclosure.
FIG. 3 is a flowchart for explaining a method of learning an AI model according to an embodiment of the present disclosure.
FIG. 4 is a block diagram for explaining a method of learning an AI model according to an embodiment of the present disclosure.
FIG. 5 is a block diagram for explaining a method of predicting the properties of a material and/or a substance or device including the material according to an embodiment of the present disclosure.
FIG. 6 is a block diagram for explaining a method of learning an AI model according to another embodiment of the present disclosure.

### DETAILED DESCRIPTION OF EXEMPLARY EMBODIMENTS

The following embodiments are provided as examples so that the spirit of the present invention may be sufficiently conveyed to those skilled in the art to which the present invention pertains. Therefore, the present invention is not limited to the embodiments described below and may be embodied in other forms.

Throughout the present disclosure, identical reference numerals refer to the same components. The present disclosure does not describe all elements of the embodiments, and general contents within the technical field to which the present invention or overlapping details between embodiments pertains are omitted. The terms "unit," "module," "member," and "block" used in the specification may be implemented in either software or hardware, and according to embodiments, it is also possible for a plurality of "units," "modules," "members," and "blocks" to be implemented as a single component, or for one "unit," "module," "member," and "block" to include a plurality of components.

Throughout this specification, when a part is described as being "connected" to another part, this includes not only cases where they are directly connected, but also cases where they are indirectly connected, and an indirect connection includes a connection via a wireless communications network.

In addition, when a part is said to "include" a certain component, this means that it may further include other components rather than excluding other components, unless specifically stated to the contrary.

Throughout this specification, when a member is said to be located "on" another member, this includes not only the case where the member is in contact with the other member, but also the case where another member exists between the two members.

The terms such as first, second are used to distinguish one component from another component, and the components are not limited by the aforementioned terms.

Singular expressions include plural expressions unless the context clearly indicates otherwise.

The identification symbols in each step are used for the convenience of explanation and do not explain the order of each step, and each step may be performed in a different order than specified unless the context clearly indicates a specific order.

A system of predicting the properties of a material according to an embodiment of the present disclosure may include a device comprising all of various devices capable of performing computational processing and providing results to users. For example, the system of predicting the properties of the material according to the present disclosure may include one or more of a computer, a processor, a server device, and/or a portable terminal, or may be in any one form having the same or similar functions as these.

In addition, a system of predicting the properties of a material according to an embodiment of the present disclosure may include implementations such as a service provided from a server to a user terminal in the form of a web service, or a form in which a server and a user terminal are linked.

However, a method which is performed by a system of predicting the properties of a material according to an embodiment of the present disclosure is not limited thereto, and all types of implementable systems may be included in the present disclosure.

Here, the computer may include, for example, but not limited to, a notebook, desktop, laptop, tablet PC, slate PC, etc. equipped with a WEB Browser

[29] The server device may be a server configured to process information or data by communicating with an external device, and may include an application server, a computing server, a database server, a file server, a game server, a mail server, a proxy server, and a web server, etc, but not limited thereto.

The portable terminal is, for example, a wireless communication device that provides portability and mobility, and may include all types of handheld-based wireless communication devices such as PCS(Personal Communication System), GSM(Global System for Mobile communications), PDC(Personal Digital Cellular), PHS(Personal Handyphone System), PDA(Personal Digital Assistant), IMT(International Mobile Telecommunication)-2000, CDMA(Code Division Multiple Access)-2000, WCDMA(W-Code Division Multiple Access), WiBro(Wireless Broadband Internet) terminals, smart phones, and wearable devices such as a watch, ring, bracelet, anklet, necklace, glasses, contact lenses, or a head-mounted-device (HMD).

The AI models in some embodiments of the present disclosure may be controlled, executed, learned, or operated by at least one processor configured to perform at least one of tasks. The AI models may be stored in memory, and the feature data according to certain embodiments of the present disclosure may also be stored in the memory.

In addition, according to some embodiments of the present disclosure, command that causes the at least one processor to perform operations may be included in at least one memory. At least one processor may cause an AI model to operate, and may also cause other components (e.g., a transformation unit, a calculation unit, etc.) that implement the system to operate in addition to the AI model.

In addition, in certain embodiments of the present disclosure, the AI model may include a neural network, a machine learning model, etc. However, the present invention is not limited thereto.

Hereinafter, exemplary embodiments of the present disclosure will be described in detail with reference to the accompanying drawings.

The present disclosure generally relates to a system, method, and program for predicting the properties of a material having a multi-phase, and more specifically, to a system, method, and program for predicting the properties of a material having a multi-phase using artificial intelligence.

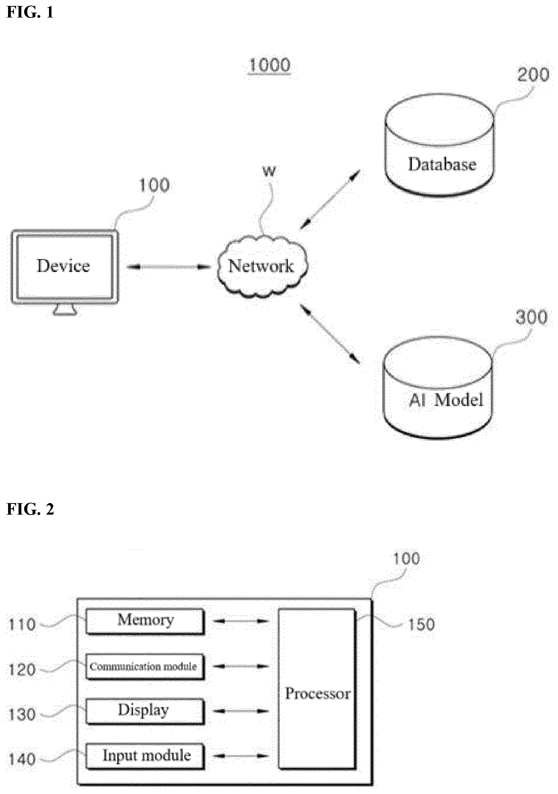

FIG. 1 is a schematic diagram of a system in which a method of predicting the properties of a material is implemented according to an embodiment of the present disclosure.

Referring to FIG. 1, a system 1000 may include a device 100, a database 200, and an artificial intelligence (AI) model 300.

The device 100, database 200, and AI model 300 included in the system 1000 may perform communication via a network W. For example, the network W may include a wired network and a wireless network. The network may include various networks such as a Local Area Network (LAN), a Metropolitan Area Network (MAN), and a Wide Area Network (WAN).

In addition, the network W may include the World Wide Web (WWW). However, the network W according to an embodiment of the present disclosure is not limited to the networks listed above, and may include any type of networks, for example, a known wireless data network, a known telephone network, or a known wired and/or wireless television network.

The device 100 may obtain or output information about a material, e.g., feature data about material information, based on the AI model 300. For instance, the feature data may include a feature vector as data representing the material information, and the feature value of the material information may be a negative or positive value and may include a value below the decimal point.

The material may be a material having a multi-phase. Here, the phase means a state in which a material has uniform physical properties from a macroscopic perspective under specific conditions, and the material may change phases continuously or discontinuously. The material may change phases continuously or discontinuously when the material is used. For example, the material may include a cathode material of a secondary battery, and the cathode material may include Li. In addition, the material may include a Thermally Activated Delayed fluorescence (TADF) material that may be used in an organic light emitting diodes (OLED) device. However, the material according to an embodiment of the present disclosure is not limited thereto, and the material may include any type of material that may have multi-phases.

The material information may include chemical formulas, element or atomic composition ratios, element composition ratios, physical and/or chemical information, molecular structure information, active ion information, and information about molecules that change during phase transitions, and the chemical information may include various types of molecular information (e.g., simplified molecular-input line-entry system (SMILES), international chemical identifier (INCHI), or self-referencing embedded string (SELFIES), etc.). However, the material information is not limited thereto, and may include any type of information that may distinguish differences between different materials.

In addition, the device 100 may obtain or output feature data on each phase of a material having a multi-phase (i.e., phase information) based on the AI model 300. The feature data is data representing phase information of a material and may include a feature vector, may be a negative or positive value, and may include a value below the decimal point.

The phase information of a material may include information that distinguishes the difference between each phase, information representing each phase, etc. For example, the phase information may include the composition ratio of elements or atoms, the composition ratio of elements, physical and/or chemical information, molecular structure information, etc. For example, if the material contains a Li cathode material, the Li cathode material changes the composition ratio of Li by being moved out from the cathode material in the form of active ions (Li⁺) during secondary battery charging/discharging, and thus is transformed into a different phase, and therefore, the phase information may include the Li composition ratio of the cathode material, the amount of change in the Li composition ratio, the ratio of Li to other elements other than Li (molar ratio, mass ratio, etc.), etc.

The device 100 may calculate or predict the properties of a material, the properties of a device or substance including the material based on the obtained feature data. In one embodiment, the device 100 may be used to calculate or predict the properties of a material containing a Li positive electrode material, the capacity of a lithium-ion secondary battery including the material, the average voltage, etc.

The database 200 may store various types of learning data for learning the AI model 300. In addition, the database 200 may store material information, phase information, simulation result information, etc., and in various embodiments, the database 200 may also store output data output by the AI model 300. However, the system 1000 may not include the database 200 after the learning of the AI model 300 is completed.

Fig. 1 shows an exemplary embodiment in which the database 200 is implemented as a separate device from the device 100. In this embodiment, the database 200 may be connected to the device 100 through the network W, for example, a wired and/or wireless network. However, this is only one embodiment, and the database 200 may be included in the device 100. For instance, the database 200 may be implemented as a component of the device 100.

FIG. 1 shows an exemplary embodiment in which the AI model 300 is implemented or located outside the device 100 (e.g., a cloud-based AI model), but is not limited thereto, and the AI model 300 may be included in the device 100 as a component in the device 100.

FIG. 2 is a block diagram illustrating the configuration of a device for performing a method of obtaining feature data or a device for performing a method of predicting the properties of a material having a multi-phase using artificial intelligence according to an embodiment of the present disclosure.

Referring to FIG. 2, the device 100 may include a memory 110, a communication module or communicator 120, a display 130, an input module 140, and a processor 150. However, the present invention is not limited thereto, and the device 100 may have its software and hardware configurations modified, added, and/or omitted within a range obvious to those skilled in the art for performing one or more appropriate operations according to embodiments of the present disclosure. In addition, the device 100 may be replaced with a system, and the device 100 may include a plurality of devices, and in this case, each component included in the device 100 may be included in at least one of the plurality of devices.

The memory 110 may store data supporting or performing various functions of the device 100, programs for the operation of the processor 150 and/or the execution in the processor 150, and input and/or output data, and may store multiple application programs or applications driven or executed in the device 100, data for the operation of the device 100, commands, and AI models. One or more of these application programs may be downloaded from an external server via wireless communication. The memory 110 may store commands or information that causes the processor 150 to perform operations.

This memory 110 may include any type of storage medium, for example, but not limited to, flash memory type, hard disk type, solid state disk type (SSD type), silicon disk drive type (SDD type), multimedia card micro type, card-type memory (e.g., SD, XD memory, or etc.), RAM (random access memory), static random access memory (SRAM), ROM (Read-only memory), electrically erasable programmable read-only memory (EEPROM), programmable read-only memory (PROM), magnetic memory, magnetic disk, and optical disk.

In addition, the memory 110 may be a separate device from the device 100 and may include a database connected by wire or wirelessly. The database 200 shown in FIG. 1 may be implemented as a component of the memory 110.

The communication module or communicator 120 may include one or more components configured to perform communication with an external device, and may include, for example, one or more of a broadcast receiving module, a wired communication module, a wireless communication module, a short-range communication module, or a location information module.

The wired communication module may include various wired communication modules such as a local area network (LAN) module, a wide area network (WAN) module, or a value added network (VAN) module, as well as various cable communication modules such as a universal serial bus (USB), a high definition multimedia interface (HDMI), a digital visual interface (DVI), recommended standard232 (RS-232), power line communication, or plain old telephone service (POTS).

The wireless communication module may include a wireless communication module that performs or supports various wireless communication methods such as global system for mobile communication (GSM), code division multiple access (CDMA), wideband code division multiple access (WCDMA), universal mobile telecommunications system (UMTS), time division multiple access (TDMA), long term evolution (LTE), 4G, 5G, and 6G, in addition to a WiFi module and a wireless broadband module.

The display 130 displays or outputs information or data processed in the device 100, data input or output through the AI model 300, etc. In addition, the display 130 may display execution screen information of an application program (for example, an application) driven or executed in the device 100, or user interface (UI) or graphical user interface (GUI) information according to such execution screen information.

The input module 140 may be configured to receive information from a user. When information is input through the user input unit, the processor 150 may control the operation of the device 100 in response to the input information.

For instance, the input module 140 may include hardware-type physical keys (e.g., buttons, dome switches, jog wheels, jog switches, and so on located at one or more of the front, back, or side of the device 100) and software-type touch keys. As an example, the touch keys may comprise virtual keys, soft keys, or visual keys displayed on a touchscreen type display through software processing, or include touch keys placed on a part of the device 100 other than the touchscreen. Meanwhile, the virtual keys or visual keys may be displayed on the touchscreen in various forms, and may be formed as, for example, graphics, text, icons, videos, or a combination thereof.

The processor 150 may also include a memory configured to store data for an algorithm for controlling operations of components (e.g. one or more operation of learning or executing an AI model) in the device 100 or a program configured to reproduce the algorithm, and at least one processor configured to perform the aforementioned operations using the data stored in the memory. In this exemplary embodiment, the memory and the processor may be implemented as separate chips, or may be implemented as a single chip.

In one embodiment, the system 1000 or device 100 according to an embodiment of the present disclosure may include at least one processor. And, in a case in which the system 1000 or device 100 includes a plurality of processors, the plurality of processors may be included in a plurality of different devices 100, respectively.

In addition, the processor 150 may control one or more of the components described above in combination to implement various embodiments of the present disclosure described below in the device 100.

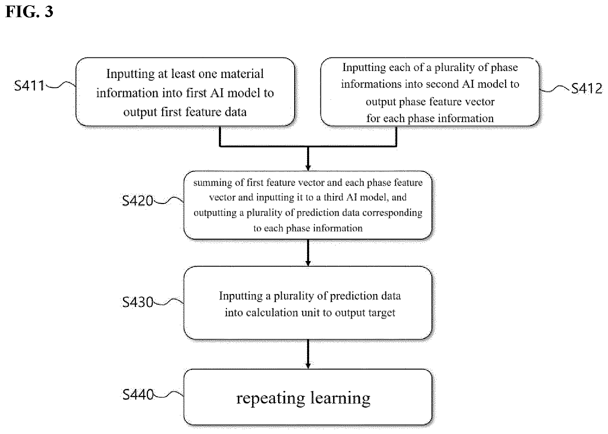

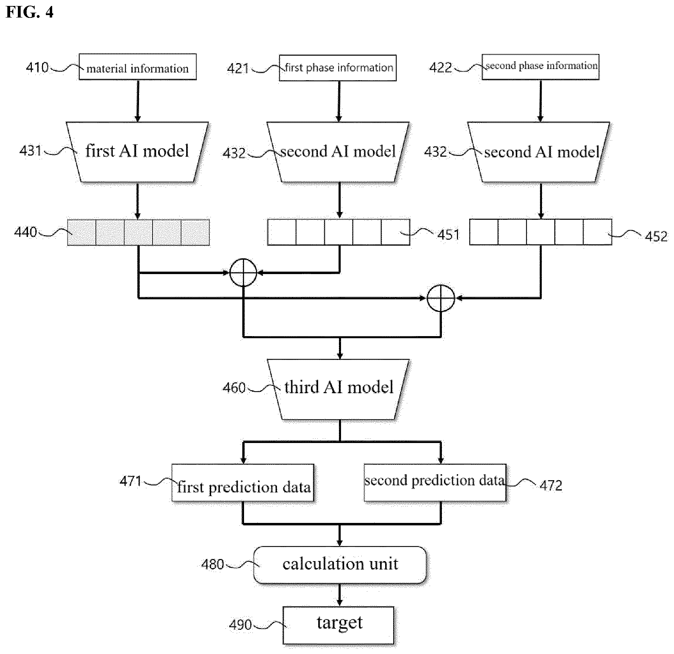

FIG. 3 is a flowchart for explaining a method for learning an AI model according to embodiments of the present disclosure, and FIG. 4 is a block diagram for explaining a method for learning an AI model according to embodiments of the present disclosure.

Referring to FIGS. 3 and 4, a method for learning an AI model according to an embodiment include inputting material information 410 into a first AI model 431 to output first feature data 440 (S411), inputting each of a plurality of first and second phase information 421 and 422 into a second AI model 432 to output a plurality of phase feature data 451 and 452 corresponding to each of the first and second phase information 421 and 422 (S412), summing operation on the first feature data 440 and each of the first and second phase feature data 451 and 452, inputting the summing result into a third AI model 460 to output a plurality of first and second prediction data 471 and 472 corresponding to each of the first and second phase information 421 and 422 (S420), inputting the plurality of first and second prediction data 471 and 472 into a calculation unit or calculator 480 to output a target 490 (S430), and repeating operations S411 to S430 described above (S440).

The method of learning an AI model according to some embodiments of the present disclosure may be performed by at least one processor. In addition, commands or information that causes one or more processors to execute or perform operations may be included in at least one memory. One or more processors or one or more memories may be included in a plurality of devices. In addition, the method of learning an AI model according to certain embodiments of the present disclosure may be performed by one or more operations performed by the command or information.

Each step of learning an AI model will be described in detail below.

First, a method of learning an AI model according to one embodiment includes inputting material information 410 into a first AI model 431 to output first feature data 440 (S411), and inputting each of a plurality of first and second image information 421 and 422 into a second AI model 432 to output a plurality of first and second image feature data 451 and 452 corresponding to each of the first and second image information 421 and 422 (S412).

For example, in S411, the material information 410 may include information or factors representing a material of which properties are to be predicted. The material information 410 may include chemical formula, composition ratio of elements or atoms, composition ratio of elements, chemical information, molecular structure information, active ion information, information on molecules that change during phase transition, etc. In the case of a Li cathode material, the material information 410 may include information on the chemical composition of the material constituting the Li cathode material and active ion (Li+) as representative.

The first AI model 431 configured to perform learning according to an embodiment of the present disclosure may be a preset algorithm for outputting feature data for the material information 410, or may be a model learned based on various information of the material (e.g. composition enthalpy, refractive index, band gap, phonon frequency, bulk modulus, Devai temperature, thermal conductivity, thermal expansion coefficient, decomposition enthalpy, etc.). The algorithm of the first AI model 410 may be multi-layer perceptron (MLP), graph neural network (GNN), transformer encoder, etc., but not limited thereto.

In one embodiment, the first feature data 440 that is output by inputting the material information 410 to the first AI model 431 may have N values (C₁, C₂, C₃...C_{N}) that are N dimensions. The first feature data 440 may include a feature vector. The first AI model 431 may vectorize the input material information 410 through element embed, fractional embed, etc., and then output the vectorized input material information as feature data through a regressor (e.g. a model configured to predict feature data from vectorized information) including multi head attention and multi-layer perceptron (MLP) or residual network, and the output feature data may be in matrix format or in a plurality of dimensions, and only some feature data may be selected and output from the entire feature data. When selecting only some feature data, the selected feature data may be a value representing the composition information of the material. However, the method in which the first AI model 431 outputs the first feature data 440 through vectorization is not limited to thereto.

The material information 410 may include factors that may be used as variables for predicting the properties of materials and devices or materials including the materials through learning by the AI model. For example, when learning an AI model for predicting the properties of a Li cathode material and a secondary battery including the Li cathode material, when the variable used for predicting the properties is the chemical composition of the material constituting the Li cathode material, the material information 410 input to the first AI model 431 may include information or factors regarding the chemical composition of the material constituting the cathode material. Accordingly, according to one embodiment, the properties according to the chemical composition of the cathode material may be predicted through the learned AI model.

In S412, the plurality of the first and second phase information 421 and 422 may include information that may distinguish the difference between each phase, information representing each phase, etc. For instance, the first and second phase information 421 and 422 may include the composition ratio of elements or atoms, the composition ratio of elements, physical/chemical information, molecular structure information, etc. For example, if the material contains a Li cathode material, the Li cathode material changes the composition ratio of Li by being moved from the cathode material in the form of active ions (Li⁺) during secondary battery charging or discharging, and then is transformed into another phase, and therefore, the phase information may include, as representative, the Li composition ratio of the cathode material, the amount of change in the Li composition ratio, and the ratio of Li to other elements other than Li (molar ratio, mass ratio, etc.).

In one embodiment, the plurality of phase information may include first phase information 421 and second phase information 422. The first and second phase information 421 and 422 includes information about the first phase and information about the second phase, respectively. The first phase and the second phase are different phases from each other. When the material has different phases, the plurality of phase information may include information about the various phases as representative factors. For example, when the material is a Li cathode material, the change in the active ion (Li⁺) causes a phase change, and during discharge, the active ion (Li⁺) is almost entirely contained in the cathode material in the form of Li, and during charge, at least a portion of the Li contained in the cathode material is released in the form of the active ion (Li⁺). In this case, the first phase information 421 and the second phase information 422 may each include information about the Li ratio during discharge and information about the Li ratio during charge, respectively. In this case, the ratio of Li may include, for example, the ratio of Li to the entire cathode material (including, but not limited to, a molar ratio, a mass ratio, etc.).

In various embodiments, the plurality of the first and second phase information 421 and 422 may be obtained from actual experimental data, simulation data, known data, etc. For example, if the material is a Li cathode material, and the first phase information 421 and the second phase information 422 are to be used as information on the Li ratio during charge or discharge, the first phase information 421 and the second phase information 422 may be obtained using data obtained through simulation. However, the present invention is not limited thereto.

Meanwhile, FIG. 4 illustrates an AI learning method using two pieces of phase information 421 and 422, but the present invention is not limited thereto, and the AI learning method according to the present invention may include inputting three or more pieces of phase information.

The second AI model 432 configured to perform learning according to an embodiment of the present disclosure may be a preset algorithm for outputting feature data for a plurality of first and second phase information 421 and 422, or may be a model learned based on multiple information on a substance. The algorithm of the second AI model 432 may be, for example, but not limited to, a multi-layer perceptron (MLP), a graph neural network (GNN), a Transformer Encoder, etc.

In one embodiment, the phase feature data 451 and 452 that is output by inputting a plurality of the first and second phase information 421 and 422 to the second AI model 432 may have N values (C₁, C₂, C₃...C_{N}) that are N-dimensional. The phase feature data 451, 452 may include a feature vector. The plurality of the first and second phase feature data 451 and 452 correspond to the input the plurality of the first and second phase information 421 and 422, and referring to FIG. 4, the feature data corresponding to the first phase information 421 may be the first phase feature data 451, and the feature data corresponding to the second phase information 422 may be the second phase feature data 452.

In various embodiments, the operation of outputting first feature data 440 using the first AI model 431 (S411) and the operation of outputting upper feature data 451 and 452 using the second AI model 432 (S412) may be performed simultaneously or sequentially. When those operations are sequentially performed, the order for performing the operations is not limited.

Since the plurality of first phase information 421 includes information about each phase of a material having a multi-phase, when the material is used as a device, material, and so on in a form having a multi-phase, the properties of the material, and the device or substance including the material may be predicted using the AI model according to some embodiments of the present disclosure. For example, in the case of learning an AI model for predicting the properties of a Li cathode material and a secondary battery including the Li cathode material, since the Li cathode material changes a phase depending on charge or discharge, the properties of the cathode material and the secondary battery including the cathode material may be predicted using the AI model learned according to one embodiment.

In an embodiment, the first feature data 440 may include a class token form. The first feature data 440 including the class token form may be a representative vector representing factors regarding material information and a plurality of phase information. Accordingly, the first feature data 440 to which repeated learning has been performed may be utilized as a representative vector (representation) that includes information regarding what properties a material including input material information has when the has multi-phase during AI model learning.

Next, the first feature data 440 and each phase feature data 451 and 452 are added and input to the third AI model 460, and a plurality of the first and second prediction data 471 and 472 corresponding to each of the first and second phase information 421 and 422 are output (S420). The first feature data 440 may be added and input to the third AI model 460 and output as the first prediction data 471. The first feature data 440 may be added and input to the third AI model 460 and output as the second prediction data 472.

The method for learning the AI model according to some embodiments of the present disclosure may comprise one or more sum operations. The sum operation may include summing the first feature data 440 and each of the phase feature data 451 and 452. For example, if the first feature data 440 is an 8-dimensional vector and each of the phase feature data 451 and 452 is an 8-dimensional vector, the vector input to the third AI model 460 may be a 16-dimensional vector.

In one embodiment, the third AI model 460 performing learning according to an embodiment of the present disclosure may be a preset algorithm or a pre-learned model. The algorithm of the third AI model 460 may be, for example, but not limited to, a multi-layer perceptron (MLP), a graph neural network (GNN), a transformer encoder, etc.

Next, the first prediction data 471 and the second prediction data 472 are input into the calculation unit or calculator 480 to output the target 490 (S430).

The calculation unit or calculator 480 may be configured to perform various operations, or may include an AI model. The AI model may be a preset algorithm or a pre-learned model. The algorithm of the third AI model 460 may be, for instance, but not limited to, a multi-layer perceptron (MLP), a residual network, etc.

The target 490 may include information about the properties of the material and/or the properties of a substance or device including the material. For example, if the material is a Li cathode material and the model predicts the properties of a secondary battery containing the Li-based cathode material, the target 490 may include the capacity, average voltage, etc. of the secondary battery.

In one embodiment, the error ratios of the information included in the target 490 may be different or the same. The information included in the target 490 may be obtained from, for example, actual experimental data, simulation data, known data, etc., and in this case, the error ratios of the information output to the target 490 may be different. For example, the target 490 may include a first output value and a second output value, and the error ratios for the first output value and the second output value may be set to be different or the same. In another embodiment, the first output value may be a capacitor, and the second output value may be an average voltage, and in this case, the error ratio of the first output value to the second output value may be adjusted in a range of 1:9 to 9:1. In various embodiments, the error ratio of the first output value to the second output value may be any one of 9:1, 5:5, and 1:9.

Subsequently, the learning processes of S411 to S430 may be repeated (S440). In this case, the material information 410 and a plurality of phase information 421, 422 input in the repeated learning process may be input in various modified forms.

In certain embodiments of the present disclosure, the first AI model 431, the second AI model 432, and the third AI model 460 may learn using output values or output data. The parameters of the algorithm may be adjusted in various ways during the repeated learning process.

In an embodiment of the present disclosure, the first AI model 431, the second AI model 432, and the third AI model 460 may perform at least one of the tasks of execution, learning, or driving by at least one processor. In addition, one or more of the first AI model 431, the second AI model 432, or the third AI model 460 may perform at least one of the tasks of execution, learning, or driving by another processor.

According to an AI model learning method according to one embodiment of the present disclosure, the first feature data 440 includes data repeatedly learned by inputting the material information 410 and the first and second phase information 421 and 422 regarding a plurality of phases of the material into the AI model and using the output values output, and thus, the first feature data 440 may include information regarding the properties of the material according to the phase of the material. Therefore, when trying to predict the properties of the material, even when it is not known exactly how the multi-phases of the material change, the properties of the material having a multi-phase and/or the properties of the substance or device including the material may be more accurately predicted by using the first feature data 440.

In particular, compared to the conventional method of predicting the properties of materials using artificial intelligence, the conventional method assumes that there is one phase, so the properties are predicted based on the Li composition at the time of manufacturing the cathode material, and the product properties are predicted using these. Therefore, when used in an actual lithium-ion secondary battery such as a cathode material, if the Li composition changes depending on the usage status (e.g., charge or discharge), the prediction accuracy decreases if information on each phase is not entered, so when using the conventional prediction method, information on each phase is necessary for each prediction.

However, according to some embodiments of the present disclosure, since an AI model configured to learn by inputting material information and a plurality of phase information is utilized, the first feature data 440 already includes information according to the phase of the material, and therefore the first feature data 440 may be utilized as input data as a representative vector of the material without having to newly obtain and input information for each phase each time a prediction is made, thereby improving the accuracy of property prediction.

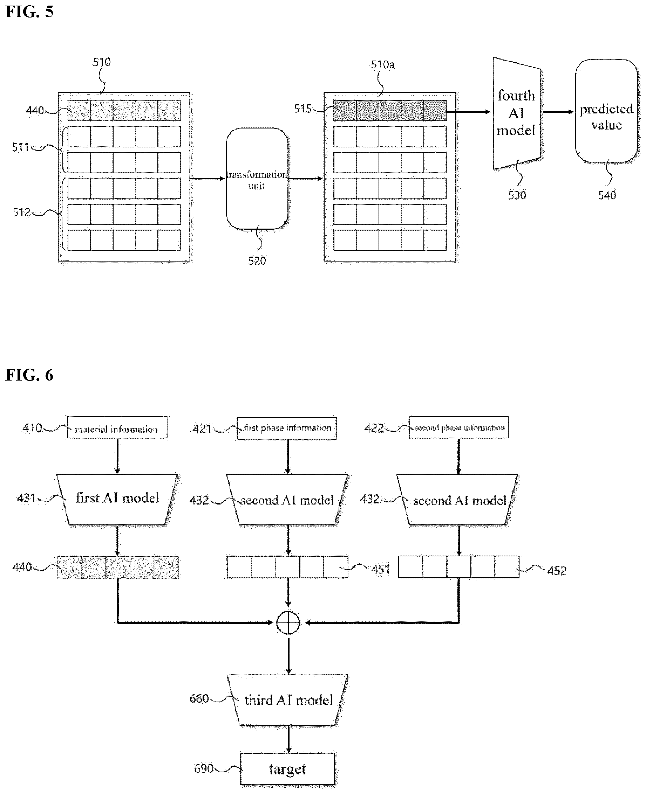

FIG. 5 is a conceptual diagram illustrating a method for predicting the properties of a material and/or the properties of a substance or device including the material according to embodiments of the present disclosure.

Referring to FIG. 5, the first feature data 440 generated according to the exemplary embodiments described with reference to FIGS. 3 and 4, and an embed 510 including at least one of other material information 511 of the material or information 512 about a substance or device including the material are input to the transformation unit 520. The embed 510 may include, for example, but not limited to, a concatenate embed.

The other material information 511 of the material may include information different from the material information 410 input in the process of generating the first feature data 440, and therefore may be different information that is distinguished from the material information 410. The other material information 511 of the material may include, for example, various analysis results such as crystal structure, crystal lattice length, length by axis of the crystal lattice, surface area, particle size distribution, and average density. The other material information 511 may include actual measurement data for the material, and may include x-ray diffraction (XRD), particle size distribution (PSD) analysis result information, etc. However, the other material information 511 is not limited thereto.

The information 512 about a substance or device including the material may include the applied current, applied voltage, usage conditions, etc. for the substance or device. For example, in a case that the material is a Li cathode material, the substance or device including the material may be a lithium-ion secondary battery, and in this case, the information 512 about a substance or device including the material may be the applied current and applied voltage. However, the information 512 about a substance or device including the material is not limited thereto, and various pieces of information may be included depending on the type of a material, substance or device including the material.

The first feature data 440, the other material information 511 of the material, and the information 512 about a substance or device including the material may be included in the embed 510, and may be concatenated, for example, within a concatenate embed.

The transformation unit 520 may perform various computational functions, and may also include an AI model. The transformation unit 520 may include a transformer encoder.

The embedding 510 input to the transformation unit 520 may be output as transformation data 510a, and the transformation data 510a may include second feature data 515.

The second feature data 515 includes information about the first feature data 440, and includes information about at least one of other material information 511 of the material and information about a substance or device including the material 512. The second feature data 515 may include a feature vector.

Next, when the second feature data 515 is input into a fourth AI model 530, a predicted value 540 is output.

In one embodiment, the fourth AI model 530 may be a preset algorithm or a pre-learned model. The algorithm of the third AI model 460 may be, for example, but not limited to, a multi-layer perceptron (MLP), a graph neural network (GNN), a transformer encoder, etc.

The predicted value 540 includes data or property values that are to be predicted among the properties of a material having a multi-phase and/or the properties of a substance or device including the material. For example, the predicted value 540 may include capacity, average voltage, etc.

A method for predicting the properties of a material and/or the properties of a substance or device including the material according to an embodiment of the present invention may use first property data 440 derived through a pre-learned AI model as input property data, and since the first property data 440 already includes information about the properties of the material in a multi-phase through the AI model learning method according to an embodiment of the present disclosure, even when predicting the properties of a material having a multi-phase, property prediction may be performed accurately even without inputting phase information.

For example, one of the development goals of a cathode material for a lithium-ion secondary battery is to improve the capacity, lifespan, etc. of the battery, and a lithium-ion secondary battery has the property of having a multi-phase due to changes in the amount of lithium in the cathode material during charging or discharging. According to an embodiment of the present disclosure, by inputting the chemical composition of the cathode material as material information (information as a representative state) and the phase information of the cathode material in the charge or discharge state, a feature data (utilized as a representation) including information on the above material information and phase information may be secured through an AI model learned, and by using the feature data, the properties (e.g., capacity, lifespan) of a lithium-ion secondary battery having a specific chemical composition may be more accurately predicted without additional phase information input.

Meanwhile, a method for predicting the properties of a material and/or the properties of a substance or device including the material according to an embodiment of the present disclosure may be performed in a separate device from a device that performs a method of learning an AI model described with reference to FIGS. 3 and 4. In this case, the first AI model 431, the second AI model 432, and the third AI model 460 may perform at least one of execution, learning, or driving by at least one first processor, and the fourth AI model 530 of FIG. 5 may perform at least one of execution, learning, or driving by at least one second processor different from the first processor.

However, the present invention is not limited thereto, and a method for learning an AI model according to some embodiments of the present disclosure and a method for predicting the properties of a material and/or the properties of a substance or device including the material may be performed in one or more devices, and may also be performed by one or more processors.

Meanwhile, in certain embodiments of the present disclosure, the cathode material of a lithium ion secondary battery is mainly explained as an example, but the present invention is not limited thereto, and the present invention is applicable to various materials having a multi-phase, including OLED materials.

FIG. 6 is a block diagram illustrating a method of learning an AI model according to other embodiments of the present disclosure.

An embodiment of FIG. 6 is different from the embodiment of FIG. 4 in that the first prediction data 471 and the second prediction data 472 are output, the input of the first prediction data 471 and the second prediction data 472 to the calculation unit 480 is omitted, and the first feature data 440 and the plurality of phase feature data 451, 452 are all summed together and input to the third AI model 460.

Referring to FIG. 6, the first feature data 440 and a plurality of phase feature data 451 and 452 are all summed together, and the calculation unit 480 of FIG. 4 is omitted in the embodiment of FIG. 6, thereby simplifying the AI learning.

Detailed descriptions that overlap with those described with reference to FIGS. 3 and 4 below are omitted herein.

The following experimental examples explain the prediction accuracy of a system and method for predicting properties of a material having a multi-phase using artificial intelligence according to an embodiment of the present disclosure.

The table below is a table comparing the prediction accuracy performance of models according to one embodiment of the present disclosure and models according to comparative examples. The following Example 1 and Comparative Examples are the results of prediction accuracy experiments on the properties of Li cathode materials, and

Example 1 predicted the capacity and average voltage from the battery simulation data, using the model according to the embodiment of FIG. 6, and the input information types and usage models of the comparative examples are as follows. As comparative examples, a prediction model based on reduced engineered representation (ACS Applied Materials & Interfaces, 13(45):53355?53362, 2021.), which predicts by condensing information that may be obtained from elements, and a model based on CrabNet (Npj Computational Materials, 7(1):77, 2021) that may obtain expression information from the composition of substance were used. The lower the mean absolute error (MAE) value, the better the prediction performance, and the higher the R2 score value, the higher the prediction accuracy.

**[Table 1]**

| **Section** | **Input Type** | **Model** | **MAE** | **R2 score** |
|---|---|---|---|---|
| Embodiment 1 | Chemical Composition | FIG. 6 | 0.377±0.012 | 0.570±0.041 |
| Comparative Example 1 | Chemical Composition | reduced engineered representation-based | 0.505±0.015 | 0.502±0.030 |
| Comparative Example 2 | Chemical Composition | CrabNet-based prediction model | 0.389±0.012 | 0.539±0.044 |

As shown in Table 1, the accuracy of predicting material properties is improved when using the prediction model according to an embodiment of the present disclosure compared to the existing models.

Example 2 predicted the capacity from the battery experimental data, using the model according to the embodiment of FIG. 5, and the input information types and usage models of the comparative examples are as follows. As comparative examples, a model based on CrabNet (Npj Computational Materials, 7(1):77, 2021) that may obtain expression information from the composition of the material was used. The lower the mean absolute error (MAE) value, the better the prediction performance, and the higher the R2 score value, the higher the prediction accuracy.

**[Table 2]**

| **Section** | **Input Type** | **Model** | **MAE** | **R2 score** |
|---|---|---|---|---|
| Embodiment 1 | Chemical Composition | FIG. 5+reference No. 440 | 12.67±0.85 | 0.711±0.048 |
| Comparative Example 2 | Chemical Composition | FIG. 5+CrabNet-based predictionon | 13.63±1.28 | 0.646±0.124 |

As shown in Table 2, the accuracy of predicting material properties is improved when using the prediction model according to an embodiment of the present disclosure compared to the existing model.

Meanwhile, a method for learning an AI model and a method for predicting the properties of a material and/or the properties of a substance or device including the material according to some embodiments of the present disclosure may be implemented by the system described with reference to FIGS. 1 and 2.

In addition, certain disclosed embodiments may be implemented in the form of a recording medium storing executable commands by a computer. The commands may be stored in the form of program code, and when executed by a processor, a program module may be generated to perform the operations of the disclosed embodiments. The recording medium may be implemented as a computer-readable recording medium.

The computer-readable recording medium includes all types of recording media in which instructions that may be interpreted by a computer are stored. For example, ROM (Read Only Memory), RAM (Random Access Memory), magnetic tapes, magnetic disks, flash memory, optical data storage devices and the likes may be included.

The disclosed embodiments have been described with reference to the accompanying drawings as above. It will be understood by those skilled in the art to which the present disclosure pertains that the present disclosure may be practiced in other forms than the disclosed embodiments, without changing the technical spirit or essential features of the present disclosure. The disclosed embodiments are illustrative and should not be construed as limiting.

## Claims

1. A system for implementing an artificial intelligence (AI) model for predicting properties of a material having a multi-phase, the system comprising:
memory configured to store instructions that are executable; and
one or more processors configured to execute the instructions to perform operations comprising:
inputting first material information, which comprises information regarding the material having the multi-phase including first and second phases, into a first AI model to output first feature data;
inputting first phase information, which comprises information regarding the first phase of the material, into a second AI model to output first phase feature data; and
inputting second phase information, which comprises information regarding the second phase of the material different from the first phase, into the second AI model to output second phase feature data; and
wherein the first feature data comprises information regarding the properties of the material according to the multi-phase of the material.

2. The system of claim 1, wherein:
the operations performed by the one or more processors further comprise inputting the first feature data, the first phase feature data, and the second phase feature data into a third AI model.

3. The system of claim 2, wherein:
the operations performed by the one or more processors further comprise:
summing the first phase feature data with the first feature data and inputting a summed result of the first phase feature data and the first feature data into the third AI model to output first prediction data; and
summing the second phase feature data with the first feature data and inputting a summed result of the second phase feature data and the first feature data into the third AI model to output second prediction data.

4. The system of claim 3, wherein the operations performed by the one or more processors further comprise receiving the first and second prediction data and outputting a target in response to the first and second prediction data.

5. The system of claim 2, wherein:
the operations performed by the one or more processors further comprise summing the first feature data, the first phase feature data, and the second phase feature data and inputting a summed result of the first feature data, the first phase feature data, and the second phase feature data into the third AI model.

6. The system of claim 1, wherein:
the first feature data comprises a class token form.

7. The system of claim 1,
wherein the operations performed by the one or more processors further comprise receiving concatenated embed and outputting transformed data,
wherein the concatenated embed comprises:
the first feature data;
second material information comprising information regarding the material; and
at least one of information regarding a substance or a device comprising the material, and
wherein the second material information is different from the first material information.

8. The system of claim 7, wherein:
the transformed data comprises second feature data,
the second feature data comprises information regarding the first feature data, at least one of the second material information, and the information regarding the substance or the device comprising the material.

9. The system of claim 8, wherein:
the operations performed by the one or more processors further comprise inputting the transformed data into a fourth AI model to output a predicted value.

10. The system of claim 9, wherein:
the predicted value comprises a property value of the substance or the device comprising the material.

11. The system of claim 1, wherein:
at least one of the first feature data or the second feature data is a feature vector.

12. A computerized method comprising:
inputting first material information, which comprises information regarding a material having a multi-phase including first and second phases, into a first AI model to output first feature data;
inputting first phase information, which comprises information regarding the first phase of the material, into a second AI model to output first phase feature data; and
inputting second phase information, which comprises information regarding the second phase of the material different from the first phase, into the second AI model to output second phase feature data,
wherein the first AI model and second AI model are executed or learned by one or more processors, and
wherein the first feature data comprises information regarding properties of the material according to the multi-phase of the material.

13. The computerized method of claim 12, further comprising:
inputting the first feature data, the first phase feature data, and the second phase feature data into a third AI model.

14. The computerized method of claim 12, further comprising:
outputting transformed data in response to input concatenated embed,
wherein the concatenated embed comprise:
the first feature data;
second material information comprising information regarding the material; and
at least one of information regarding a substance or a device comprising the material, and
wherein the second material information is different from the first material information.

15. A program stored on a non-transitory computer-readable recording medium storing instructions that are executable by one or more processors to perform operations included in the computerized method according to claim 12.

16. A system for implementing an AI model for predicting properties of a material having a multi-phase, the system comprising:
memory configured to store instructions that are executable; and
one or more processors configured to execute the instructions to perform operations comprising:
outputting transformed data by inputting an embed which comprises feature data regarding the material, material information of the material, and at least one of information regarding a substance or a device comprising the material; and
inputting the transformed data into an AI model to output a predicted value,
wherein the feature data comprises information regarding the properties of the material according to the multi-phase of the material.
